(19) Europäisches Patentamt

European Patent Office

Office européen des brevets

(11) **EP 1 199 353 A1**

(12) **EUROPEAN PATENT APPLICATION**

(43) Date of publication:
   **24.04.2002 Bulletin 2002/17**

(51) Int Cl.[7]: **C12N 1/06**

(21) Application number: **01203706.5**

(22) Date of filing: **01.10.2001**

(84) Designated Contracting States:
   **AT BE CH CY DE DK ES FI FR GB GR IE IT LI LU
   MC NL PT SE TR**
   Designated Extension States:
   **AL LT LV MK RO SI**

(30) Priority: **10.10.2000 DE 10050088**

(71) Applicants:
   • **Bestfoods
     Englewood Cliffs, NJ 07632 (US)**
     Designated Contracting States:
     **BE CH DK ES FI FR GB GR IE IT LI NL PT SE TR
     AT**
   • **Bestfoods Deutschland GmbH & Co. OHG
     74074 Heilbronn (DE)**
     Designated Contracting States:
     **DE**

(72) Inventors:
   • **Mönch, Susanne, Bestfoods Deutschland
     74074 Heilbronn (DE)**
   • **Stute, Rolf
     71686 Remseck, Neckarems (DE)**

(74) Representative: **Wurfbain, Gilles L. et al
   Unilever N.V.,
   Patent Department,
   P.O. Box 137
   3130 AC Vlaardingen (NL)**

(54) **Process for producing yeast extracts**

(57)    The invention relates to a process for producing yeast extracts by treating yeast suspensions or yeast pastes and separating off the insoluble constituents, in which the yeast suspensions or yeast pastes are subjected to high-voltage electrical pulses.

## Description

Yeast extracts, together with meat extract, soy sauces, fish sauces and seasonings produced by hydrolysis with hydrochloric acid are important flavouring ingredients in the food industry. They are used alone, or frequently in combination with addition of other flavouring and/or flavour-enhancing components, for example salt, sugars, sodium glutamate.

Yeast extracts are, in addition, the amino-nitrogen-containing reaction partner in reaction flavourings which are produced, with addition of a sugar component, via the known Maillard reaction.

In this manner, yeast extracts are a constituent of numerous foods. Similarly, they are used in animal nutrition.

Because of the high content of free amino acids having a similar composition to meat extract, yeast extracts were also used in medicine in the so-called amino acid therapy introduced by Abderhalden.

The use of yeast extracts in foods is of considerable importance as taste-producing and flavouring ingredient.

The use of yeast extracts is also of great importance for nutrient media in microbiology and biotechnology, where they replace the meat broth introduced by L. Pasteur as nutrient medium.

In the production of yeast extracts, the composition of the extracts and the extract yield are of critical importance. Yeast extracts here, in the strict sense, are not extracts, that is to say products produced solely by extraction, at all.

Virtually nothing can be extracted from living yeast cells, in which the cell wall alone makes up about 1/3 of the dry matter. In principle this is not achieved until after cell death, in which, however, the endogenous enzyme systems must not be inactivated. The processes used in this sense for producing yeast extracts are autolysis and plasmolysis. However, processes are also known in which the endogenous yeast enzymes are completely inactivated and the extracts are obtained by added enzymes such as proteases and glucanases which break down cell wall.

A process of this type is described by EP 354 610 A1, in which, by breaking down the yeast, firstly with proteolytic enzymes under anaerobic conditions, and then with RNA-cleaving enzymes under oxidizing conditions, 5'-ribonucleotides especially are obtained.

Autolysis can begin in a natural manner at the end of the stationary growth phase. Autolysis can be promoted by physical inducers such as by changing the temperature, changing the osmotic pressure (plasmolysis), irradiation with x-rays and mechanical comminution, or by chemical inducers (Babayan et al.: "Autolysis in yeasts" Acta Biotechnol. 5 (1985) 2, 129-136). However, it is generally induced by mild heating to 45-60°C or induced in the case of plasmolysis by addition of sodium chloride or calcium chloride. The endogenous cell enzymes which are not inactivated in this manner, especially proteases, nucleases and glucanases, cause a cleavage of the proteins, carbohydrates and nucleic acids present in the yeasts to give oligopeptides, amino acids, nucleotides and sugars. The products obtained without separating off the cell wall fragments are termed autolysates, and the products obtained after separation are termed extracts.

To increase the yield, generally what are termed "autolysis promoters" are added, for example ethyl acetate, isopropanol, chitosan or detergents. Added enzymes, in particular proteases (for example pepsin, pancreatin) also increase the yield, as also does mechanical digestion of the cells, for example by highpressure homogenization. Each of these processes increases the extract yield, but also changes the composition of the soluble substances in the extract.

The yeasts used are primarily baker's yeast and brewer's yeast. In principle, however, all yeasts are suitable. Depending on the type of yeast used and the production process employed, yeast extracts differ in taste and colour. What is wanted is generally a mild, meat-broth-like taste and flavour.

To ensure this, complete removal even of the very fine insoluble constituents after autolysis is required, with, in addition to centrifuges and separators, filters and filter aids frequently additionally having to be used.

A process which is specially orientated towards increasing the flavour-enhancing action uses the enzyme 5'-phosphodiesterase to cleave the RNA released during autolysis (US Patent 4303680). Of the 5'-nucleotides obtained in this manner, however, only the 5'-guanosyl monophosphate (5'-GMP) and 5'-inosine monophosphate (5'-IMP) have a flavour-enhancing action, however, but not, in contrast, 5'-adenosyl monophosphate (5'-AMP). Therefore, in a variant of the above process, an AMP deaminase is used so that from the 5'-AMP obtained initially, 5'-IMP is then formed (Lee et al., Korean Journal of Applied Microbiology and Bioengineering, 21(3), 276-280, 1993).

Suitable yeasts are, for example, Saccharomyces species, for example Saccharomyces cerevisiae strain yUR470094, deposited at the Central Bureau voor Schimmelcultures, Baarn, the Netherlands, under the number CBS 270.89; in addition, Kluyveromyces species, Candida species, Torula species, Fusarium species, Zymomonas species and Pychia species, in particular Saccharomyces cerevisiae, Kluyveromyces lactis, Kluyveromyces fragilis, Kluyveromyces marxianus, Candida utilis.

The object underlying the invention is to provide a further, readily useable process which is completely different from the processes known hitherto, a process by which yeast extracts may be produced without the autolysis or plasmolysis otherwise required or the yields in autolysis can be improved, but in particular to provide yeast extracts having a quite different composition and different profile of properties.

According to the invention it is proposed that the yeast suspensions or yeast pastes are subjected to high-voltage electrical pulses.

Treatment with high-voltage electrical pulses is known in principle.

The generation of electric pulses and the relevant equipment required and the basic principle of electroporation with the resultant membrane permeabilization are described by Barsotti et al. (Food Review International, 15, 2, 1999 163-180; 181-213).

Under the name "Electrotransformation" or "Electroporation", this method is used in genetic engineering for transferring plasmids into microorganisms. In recent years, this technique has been increasingly studied with respect to the destruction of microorganisms as a gentle non-thermal pasteurization process.

Thus Heinze et al., in Food Biotechnology 13(2), 155-168 (1999) report on the inactivation of Bacillus subtilis at room temperature. In Wouters et al., Food Biotechnology 11(3), 193-229 (1997) there is a summary of the effects of individual electrical parameters (field strength, pulse width, pulse frequency, number of pulses) and the effect of the medium (composition, conductivity, etc.) on the destruction of various microorganisms. A survey of the effect of electrical pulses on various microorganisms, including yeasts, may be found in Barbosa-Canovas et al., ("PEF Inactivation of Vegetative Cells, Spores, Enzymes in Foods" in Preservation of Foods with Pulsed Electric Fields, Academic Press, New York, 1999) and in "Kinetics of Microbial Inactivation for Alternative Food Processing Technology": (http://vm. cfsan.fda.gov/~comm/ June 2nd, 2000).

According to Angersbach, Heinz and Knorr "Elektrische Leitfähigkeit als Mali des Zellaufschlußgrades von zellulären Materialien durch Verarbeitungsprozesse" [Electrical conductivity as an indication of degree of cell digestion of cellular materials by processing methods] (42 (1997) 4 LVT 195-200) the measurement of the electrical conductivity is suitable for quantifying cell damage in biological material as occurs during the handling and processing of fruit, vegetables, meat or yeast suspension, for example after mechanical loading (pressing, comminution, etc.), freezing, blanching, increasing ripeness, etc. The studies by Geulen (including: "Zellaufschluß durch elektrische Hochspannungspulse" [Cell digestion by high-voltage electrical pulses] ZFL 45/1994) No. 7/8, 24-27) show that the juice yield in the case of pressed carrot juices can be improved by a treatment with high-voltage electrical pulses.

Hitherto there has been no knowledge as to whether and to what extent the results of these studies can be used in practice, in particular in the production of yeast extracts.

In the inventive process, a yeast suspension is treated with high-voltage pulses, below termed briefly the electrical pulse treatment. It has been found that the yeast cells are not destroyed by this treatment, but it is assumed that, by the high-voltage pulses, essentially the membranes up to one energy input are initially reversibly made permeable, and at a correspondingly high energy input, are then also irreversibly denatured.

The degree of permeabilization can be determined via what is termed the Zp value, which can be determined by measuring the conductivity at high and low frequency.

$$Zp = 1-b \cdot \frac{K_p, HF - K_p, NF}{K_0, HF - K_0, NF} \; ; \; b = \text{factor to take into account the temperature-dependence of conductivity}$$

K = Conductivity      HF = High frequency range (3-100 MHz)
$K_0$ = Initial sample      NF = Low frequency range (1-10 kHz)
$K_p$ = Treated sample

Electrical pulses which cause permeabilization of the membranes are high-voltage pulses in the range of kV/cm having a pulse length in the μsec to msec range.

Generally, the specific energy input is 5-150 kJ/kg, preferably 60-120 kJ/kg.

Generally, a field strength of at least 2 kV/cm is employed, preferably 6-10 kV/cm.

Generally, pulse widths in the nsec-msec range are employed, preferably 0.1-0.6 msec, at a pulse number of 1-100 pulses, preferably from 10 to 50 pulses.

The yeast suspension or yeast paste used generally has a dry matter content of 15-40%, preferably 20-30% by weight.

By means of the inventive process, without employing autolysis or plasmolysis, extracts can be produced which are distinguished from known extracts by a different composition and different properties.

However, the inventive process can preferably be connected upstream of the known autolysis or plasmolysis, or used simultaneously with them. In this case very high extract yields are achieved even without autolysis promoters, although autolysis promoters can be used conjointly as is conventional. The operation without autolysis promoters, however, has the advantage that removal of the solvents used herefor can be omitted and no additives such as chitosan remain in the extract.

Enzyme additions are not necessary, but can be used in a known manner and with the same results.

The energy introduced by the high-voltage pulses is not lost, but the resultant temperature increase can be used for the subsequent autolysis, or the energy input can be controlled in such a manner that the autolysis temperature is achieved by the high-voltage pulse treatment.

The autolysis is generally carried out at a temperature of 30-75°C, preferably 40-60°C. The elevated temperature also improves the filterability of the extract.

The high-voltage pulse process used according to the invention does not imply an additional process step which is time-consuming and requires complex equipment, but can be integrated without any important change into existing processes.

The particular importance of the inventive process is not only the increase in yield, but in particular the fact that by means of this process the chemical composition of the extract and thus the taste and profile of properties can be altered overall. This finding is completely surprising and is not explained solely by the change in permeability of the cell walls. Thus by employing the electrical pulse process, yeast extracts having a novel composition and novel profile of taste and properties can be obtained.

The examples below describe the invention and show the effects to be achieved by the inventive measures, that is to say

- that a yield sufficient for extract production can be achieved without corresponding autolysis, using only the electrical pulse treatment (Example 1)

- that, in combination with autolysis, even at low Zp values an increase in yield can be achieved (Example 2)

- that when the pulse treatment is used with subsequent autolysis, 1.) even after relatively short times relatively high yields and 2.) a higher yield overall is achieved (Examples 4 and 5)

- that the direct extraction after the electrical pulse treatment gives extracts of completely different composition compared with those produced in a conventional manner by autolysis (Example 3).

- that the extraction residues after the electrical pulse treatment can be subjected in a customary manner to an autolysis and give extracts of the known composition (Example 3).

**EXAMPLES**

Example 1

500 ml of a suspension of baker's yeast (Saccharomyces cerevisiae) in water having a DM content of 21% were subjected to an electrical pulse treatment under 4 different conditions:

|  | kV/cm | Hz (1/sec) | Pulse number | Energy input kJ/kg |
|---|---|---|---|---|
| 1. | 0 | 0 | 0 | 0 |
| 2. | 6.34 | 1 | 1 | 1.86 |
| 3. | 4.99 | 1 | 10 | 11.75 |
| 4. | 6.34 | 1 | 5 | 9.3 |
| 5. | 7.12 | 1 | 40 | 87.2 |

Under these conditions, Zp values of 0.23, 0.43, 0.60 and 0.74 were achieved.

After the treatment, the suspension was centrifuged, the clear supernatant was separated off, the residue was washed 2× with water and the extract yield was determined in the combined clear solutions (drying cabinet method, quartz sand, 105°C, overnight).

Table 1 below shows that the extract yield (extract dry matter based on yeast dry matter used) increases with increasing Zp values.

| Zp | Yield [%] | Yield increase |
|---|---|---|
| Untreated | 0.78 | - |
| 0.23 | 1.47 | 1.9× |

(continued)

| Zp | Yield [%] | Yield increase |
|------|-----------|----------------|
| 0.43 | 2.47 | 3.2× |
| 0.60 | 4.26 | 5.5× |
| 0.74 | 5.78 | 7.4× |

The protein content of the extract (N×6.25) was 29.4%. The free amino acid content was 16% and that of 5'-nucleotides 13.5 g/kg (of which 5'-GMP and 5'-IMP 4.7%).
The example shows that the extract yield can be increased to more than 7 fold by a preceding treatment with high-voltage pulses.

Example 2:

A suspension of baker's yeast was treated in the same manner and under the same conditions with high-voltage pulses, but was then subjected to an autolysis without autolysis promoters (24 h at 50°C). Separation of the extract and yield determination were performed as in Example 1.
Table 2 below shows, firstly, that in this case also, higher extract yields are obtained with increasing Zp values, but, especially, that in combination with the autolysis, even at low Zp values, a marked increase in yield is achieved.

| Zp | Yield [%] | Yield increase |
|-----------|-----------|----------------|
| Untreated | 21.6 | - |
| 0.23 | 33.5 | 60% |
| 0.43 | 31.2 | 48% |
| 0.60 | 34.4 | 60% |
| 0.74 | 37.4 | 76% |

The protein content of the extract was 60%, the free amino acid content 31.1%. The 5'-nucleotide content was 4.9 g/kg.

Example 3:

Suspensions of baker's yeast were subjected to an electrical pulse treatment at 8.7 kV/cm and 20 pulses or a total energy input of 70.4 kJ/kg and were then divided into two. One part was subjected to an autolysis (24 h, 50°C) without additional promoters. The other part was refrigerated, the supernatant separated off and the centrifuge residue was extracted again by addition of water, and the extract obtained after centrifuging again was combined with the first extract. The residue thus extracted was subjected to an autolysis in the same manner as the suspension. The extracts from the autolysis of suspension and residue were in turn produced by centrifuging off the insoluble residue.
Table 3 below shows the resultant yields of dry matter (DM), protein, ash and free amino acids:

Table 3

| Extract yield and content of ash, protein (N × 6.25), free amino acids and 5'-nucleotides (AMP, GMP, IMP) in the extract after an electrical pulse treatment (8.7 kV/cm, 20 pulses, energy input 70.4 kJ/kg) | | | |
|---|---|---|---|
| | Direct extraction | Direct autolysis | Residue autolysis |
| DM yield [%] | 7.8 | 43.4 | 40.7 |
| Content of Protein [%] | 29.4 | 60.6 | 75.0 |
| Ash [%] | 19.3 | 8.0 | 6.2 |
| Free amino acids [%] | 15.9 | 31.1 | 30.8 |
| 5'-Nucleotides [mg/100 g] | 89 | 45 | 51 |

Tables 4 and 5 show the characteristic differences between the 3 extracts.

Table 4

| Composition of the free amino acids in the extracts after the electrical pulse treatment (8.7 kV/cm, 20 pulses, energy input 70.4 kJ/kg) | | | | | |
|---|---|---|---|---|---|
| Direct extraction | | Residue autolysis | | Direct autolysis | |
| Amino acid | % | Amino acid | % | Amino acid | % |
| Glutamic acid | 19.6 | Leucine | 11.3 | Leucine | 10.1 |
| | 18.8 | Valine | 7.5 | Alanine | 7.9 |
| Ornithine | 11.3 | Alanine | 7.4 | Glutamic acid | 6.9 |
| Alanine | | Phenylalanine | 7.0 | Valine | 6.9 |
| | 49.7 | Isoleucine | 6.5 | Phenylalanine | 6.3 |
| | | Glutamic acid | 6.2 | Aspartic acid | 5.8 |
| | | Lysine | 5.5 | Isoleucine | 5.8 |
| | | | 51.4 | | 49.7 |

In the extract produced directly after the electrical pulse treatment, the 3 amino acids glutamic acid, ornithine and alanine dominate; in particular, glutamic acid, which acts as a flavour-enhancer, is the main constituent. In contrast, in the autolysis, other amino acids are released, more precisely from the insoluble constituents of the yeast cell, as shown by the composition of the autolysate obtained from the centrifuge residue (Table 4).

The composition of the nucleotides also differs (Table 5).

Whereas, in the extract directly after the electrical pulse treatment, the 5'-nucleotides dominate, including the 5'-AMP as main constituent to be converted into the flavour-enhancing nucleotide 5'-IMP using the enzyme 5-AMP deaminase, 3'-nucleotides are more intensively released from the insoluble fraction of the yeast, which then dominate in the conventionally produced autolysate.

Table 5:

| Composition of the nucleotides in the extract after the electrical pulse treatment (8.7 kV/cm, 20 pulses, energy input 70.4 kJ/kg) | | | |
|---|---|---|---|
| Nucleotides | Direct extraction [mg/kg of extract] | Residue autolysis [mg/kg of extract] | Direct autolysis [mg/kg of extract] |
| 5'AMP | 7300 | 3400 | 3200 |
| 5'GMP | 490 | 60 | 90 |
| 5'UMP | 5100 | 570 | 720 |
| 3'GMP | 650 | 12 800 | 10 300 |
| 3'AMP | 530 | 7500 | 5100 |

Example 3 thus shows impressively that by using electrical pulses extracts of completely different composition, in particular those having high contents of flavour-enhancing amino acids and 5'-nucleotides, are obtained.

Example 4:

500 ml of a suspension of baker's yeast (Saccharomyces cerevisiae) in water having a DM content of 21% was subjected to an electrical pulse treatment (E = 8.9 kV/cm, 20 pulses, Q = 31.6 kJ/kg, Zp = 0.71) and were then subjected to an autolysis at 50°C.

Table 6:

| Time-dependency of yield after the electrical pulse treatment | | | |
|---|---|---|---|
| | 0 h autolysis | 5 h autolysis | 24 h autolysis |
| 31.6 kJ/kg | 9.0 | 34.1 | 41.2 |
| Control | 0.7 | 20.4 | 35.5 |

Example 5:

500 ml of a suspension of baker's yeast (Saccharomyces cerevisiae) in water having a DM content of 21% was subjected to an electrical pulse treatment (E = 6.5 kV/cm, 20 pulses, Q = 26.5 kJ/kg, Zp = 0.75), but the pH was kept constant by adding 30% strength sodium hydroxide solution.

Table 7:

| Time-dependency of the yield after the electrical pulse treatment at a constant pH of 6.6 | | | |
|---|---|---|---|
| | 0 h autolysis | 5 h autolysis | 24 h autolysis |
| 26.5 kJ/kg | 8.7 | 11.2 | 18.5 |
| Control | 1.0 | 4.6 | 14.5 |

Examples 4 and 5 show that firstly after a relatively short time and 2.) an overall higher yield is achieved by an electrical pulse treatment provided before the autolysis.

**Claims**

1. Process for producing yeast extracts by treating yeast suspensions or yeast pastes and separating off the insoluble constituents, **characterized in that** the yeast suspensions or yeast pastes are subjected to high-voltage electrical pulses.

2. Process according to Claim 1, **characterized in that** the specific energy input is 5-150 kJ/kg, preferably 60-120 kJ/kg.

3. Process according to either of the preceding claims, **characterized in that** a field strength of at least 2 kV/cm is employed, preferably 6-10 kV/cm.

4. Process according to one of the preceding claims, **characterized in that** a pulse width of 0.1-0.6 msec is employed.

5. Process according to one of the preceding claims, **characterized in that** a pulse number of 1-100 pulses is employed, preferably 10-50 pulses.

6. Process according to one of the preceding claims, **characterized in that** a yeast suspension having a dry matter content of 15-40% by weight is used, preferably 20-30% by weight.

7. Process according to one of the preceding claims, **characterized in that**, subsequently to the treatment with high-voltage electrical pulses, the suspension is subjected to an autolysis.

8. Process according to Claim 7, **characterized in that** the autolysis is carried out at a temperature of 30-75°C, preferably 40-60°C.

9. Process according to Claim 7 or 8, **characterized in that** autolysis promoters and/or enzymes are added.

European Patent
Office

**EUROPEAN SEARCH REPORT**

Application Number

EP 01 20 3706

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GANEVA V ET AL: "ELECTROPULSATION AS AN ALTERNATIVE METHOD FOR PROTEIN EXTRACTION FROM YEAST" FEMS MICROBIOLOGY LETTERS, AMSTERDAM, NL, vol. 174, no. 2, 1999, pages 279-284, XP001010438 ISSN: 0378-1097 * page 280, left-hand column, paragraph 3 * * page 284, last paragraph * | 1-9 | C12N1/06 |
| A | US 4 810 509 A (KANEGAE YUKIHIRO ET AL) 7 March 1989 (1989-03-07) * column 2, paragraph 2 * | 1-9 | |

TECHNICAL FIELDS
SEARCHED     (Int.Cl.7)

C12N

The present search report has been drawn up for all claims

| Place of search | Date of completion of the search | Examiner |
|---|---|---|
| THE HAGUE | 27 February 2002 | Mata Vicente, T. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

# EP 1 199 353 A1

## ANNEX TO THE EUROPEAN SEARCH REPORT
## ON EUROPEAN PATENT APPLICATION NO.

EP 01 20 3706

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2002

| Patent document cited in search report | | Publication date | Patent family member(s) | | Publication date |
|---|---|---|---|---|---|
| US 4810509 | A | 07-03-1989 | AT | 86297 T | 15-03-1993 |
| | | | CA | 1308297 A1 | 06-10-1992 |
| | | | DE | 3784379 D1 | 08-04-1993 |
| | | | DE | 3784379 T2 | 17-06-1993 |
| | | | EP | 0249435 A2 | 16-12-1987 |
| | | | JP | 63112965 A | 18-05-1988 |

EPO FORM P0459

For more details about this annex : see Official Journal of the European Patent Office, No. 12/82

9